(19) Europäisches Patentamt / European Patent Office / Office européen des brevets

(11)  **EP 3 008 471 B1**

(12)  **EUROPEAN PATENT SPECIFICATION**

(45) Date of publication and mention
of the grant of the patent:
**15.03.2017   Bulletin 2017/11**

(51) Int Cl.:
*G01N 33/70* [(2006.01)]

(21) Application number: **14737418.5**

(22) Date of filing: **12.06.2014**

(86) International application number:
**PCT/US2014/042186**

(87) International publication number:
**WO 2014/201291 (18.12.2014 Gazette 2014/51)**

(54)  **METHODS FOR DETERMINING TOTAL BODY SKELETAL MUSCLE MASS**

VERFAHREN ZUR BESTIMMUNG DER GESAMTKÖRPERSKELETTMUSKELMASSE

PROCÉDÉS DE DÉTERMINATION DE LA MASSE MUSCULAIRE SQUELETTIQUE TOTALE DU CORPS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **12.06.2013   US 201361834267 P
08.10.2013   US 201361888105 P**

(43) Date of publication of application:
**20.04.2016   Bulletin 2016/16**

(73) Proprietors:
• **The Regents of the University of California
Oakland, CA 94607 (US)**
• **GlaxoSmithKline Intellectual Property (No. 2)
Limited
Brentford,  Middlesex TW8 9GS (GB)**

(72) Inventors:
• **HELLERSTEIN, Marc, K.
Kensington, CA 94708 (US)**
• **EVANS, William
Emeryville, CA 94608 (US)**

(74) Representative: **Zacco Sweden AB
P.O. Box 5581
114 85 Stockholm (SE)**

(56) References cited:
• S. A. STIMPSON ET AL: "Total-body creatine pool size and skeletal muscle mass determination by creatine-(methyl-d3) dilution in rats", JOURNAL OF APPLIED PHYSIOLOGY, vol. 112, no. 11, 15 March 2012 (2012-03-15), pages 1940-1948, XP055140842, ISSN: 8750-7587, DOI: 10.1152/japplphysiol.00122.2012
• STEPHEN A STIMPSON ET AL: "Longitudinal determination of total body creatine pool size and skeletal muscle mass in rats by D3-creatine dilution", THE FASEB JOURNAL, vol. 27, 1 April 2013 (2013-04-01), page lb410, XP055140852,
• STEPHEN A. STIMPSON ET AL: "Longitudinal changes in total body creatine pool size and skeletal muscle mass using the D3-creatine dilution method", JOURNAL OF CACHEXIA, SARCOPENIA AND MUSCLE, vol. 4, no. 3, 25 June 2013 (2013-06-25), pages 217-223, XP055140795, ISSN: 2190-5991, DOI: 10.1007/s13539-013-0110-1
• CLARK RICHARD V ET AL: "Total body skeletal muscle mass: estimation by creatine (methyl-d3) dilution in humans.", JOURNAL OF APPLIED PHYSIOLOGY (BETHESDA, MD. : 1985) 15 JUN 2014, vol. 116, no. 12, 15 June 2014 (2014-06-15), pages 1605-1613, XP008172177, ISSN: 1522-1601

**Description**

CROSS-REFERENCES TO RELATED APPLICATIONS

This application claims the benefit of U.S. Prov. App. Ser. No. 61/834,267, filed June 12, 2013, and 61/888, 105, filed October 8, 2013.

FIELD OF THE INVENTION

This invention relates to methods for determining the total body pool size of creatine and total body skeletal muscle mass in a subject by the use of an orally administered tracer dose of isotope-labeled creatine.

The present invention is defined by the appended claims. Any subject-matter identified in the application as "invention", "embodiment", "aspect", etc., which exceeds the scope of the invention as represented by the claims does not form part the claimed invention but only serves as background information to better understand the invention.

BACKGROUND OF THE INVENTION

At the present time, there is no method available to directly measure skeletal muscle mass in humans. Current methods for estimating muscle mass include computerized tomography (CT), magnetic resonance imaging (MRI), dual x-ray absorptiometry (DXA), and bioelectric impedance (BIA). These methods are expensive (CT, MRI, and DXA), have limited accuracy (BIA), and may be difficult to perform in a clinical trial with a large sample size (CT, MRI, DXA). None of these methods measure skeletal muscle mass directly (see, for example, Baracos et al. (2012) J. Parenter Enteral Nutr. 36:96-107) and each method becomes less accurate as a measure of muscle mass if body water content changes (see, for example, Sarkar et al. (2005) J. Ren. Nutr. 15:152-8), a condition that is quite common in many illnesses that result in muscle wasting. Among the biochemical methods, measurement of 24 hr urinary creatinine excretion has been shown to correlate fairly well with muscle mass, consistent with the known biochemistry of creatine and creatinine. However, this method relies on the collection of all urine over a 24 hr period. While this can be done well in a specialized unit, missed samples greatly increase variability and reduce accuracy, especially in an outpatient setting.

Creatine is present almost exclusively (~98%) in skeletal muscle (Balsom et al (1994) J. Sports Med. 18:268-80. Roughly 2% of creatine is converted to creatinine per day, via an irreversible, non-enzymatic mechanism, so that ~2g per day of creatine is replaced in the whole body. Based on the assumption that conversion of creatine to creatinine is constant among and within subjects, the daily excretion rate of creatinine has been used as a metric of whole body creatine pool size (see, for example, Crim et al. (1975) J. Nutr. 105:428-38. Reviews of this method show that a relatively broad range of muscle mass per g urinary creatinine, 17-22 kg, has been used to estimate muscle mass leading to large variability in muscle mass estimates between studies. Further, there are inherent limitations to this method (in addition to the problem of making accurate 24 hr urine collections): pH and temperature affect the non-enzymatic conversion rate of creatine to creatinine; and there is degradation and metabolic removal of creatinine in the body, so that all creatinine produced is not excreted in the urine (see, for example, Wyss (2000) Physiol. Rev. 80:1107-213.

Stimpson *et al.* have described a method to measure creatine pool size and assess total body skeletal muscle mass in rodents (Stimpson et al. (2012) J. Appl. Physiol. 112:1940-8). This reference demonstrates that the enrichment of deuterated urine creatinine provided an estimate of muscle mass that was strongly correlated with independent estimates of lean mass. However, there remains a need in the art for a method to accurately determine skeletal muscle mass in human subjects regardless of age, gender, diet, and/or body composition.

BRIEF SUMMARY OF INVENTION

The present invention is based on the finding that steady-state enrichment of isotope-labeled creatinine in a urine sample following oral administration of a single defined tracer dose of isotope-labeled creatine can be used to calculate total-body creatine pool size and skeletal muscle mass in a subject.

In order to accurately determine the total-body creatine pool size of a subject according to the methods of the invention, it is necessary to determine the amount of the tracer dose of isotope-labeled creatine tracer dose that is effectively delivered to the skeletal muscle of the subject. In order to calculate the amount of isotope-labeled creatine tracer dose that is effectively administered to the skeletal muscle of the subject, it is necessary to determine the amount of the administered isotope-labeled creatine tracer dose that is excreted into the urine of the subject rather than being delivered to the skeletal muscle of the subject. The inventors of the present invention have discovered that the amount of the tracer dose of isotope-labeled creatine that is excreted into the urine can vary from subject to subject, depending on the body composition and diet of the subject. The inventors have further discovered that the amount of the isotope-labeled creatine tracer dose that will be excreted into the urine is correlated with the creatine to creatinine ratio detected in a biological sample from the patient, and thus the ratio of creatine to creatinine in the biological sample from the patient can be used to accurately calculate the amount of the isotope-labeled creatine tracer dose that is effectively administered to the skeletal muscle of the subject.

Accordingly, in one aspect the invention provides a method for determining the total body skeletal muscle mass in a subject, where the method comprises the steps of:

(a) obtaining a first biological sample from the subject, wherein the first biological sample is a urine sample;
(b) determining the ratio of creatine to creatinine in the first biological sample from the subject;
(c) orally administering 20-100 mg isotope-labeled creatine or a salt or hydrate thereof to the subject;
(d) allowing the isotope-labeled creatine to reach isotopic steady state;
(e) obtaining a second biological sample from the subject,
(f) determining the concentration of creatinine and isotope-labeled creatinine in said second biological sample to thereby determine the isotope-labeled creatinine enrichment ratio in the second biological sample;
(g) using the creatine/creatinine ratio determined in step (b) to determine the amount of isotope-labeled creatine that has been effectively delivered to the skeletal muscle of the subject;
(h) calculating the total body skeletal muscle mass of the subject according to the formula:

Total body skeletal muscle mass = (amount of isotope-labeled creatine that has been effectively delivered to the skeletal muscle of the subject as determined according to step g)/[(the isotope-labeled creatinine enrichment ratio in the second biological sample as determined according to step (f) X (the creatine content of skeletal muscle (g/kg))].

In a preferred embodiment, the subject has fasted for at least 8 hours before the first biological sample is obtained according to step (a).
In one embodiment, the second biological sample is a urine sample.
In another embodiment, the second biological sample is a blood or serum sample.

BRIEF DESCRIPTION OF THE DRAWINGS

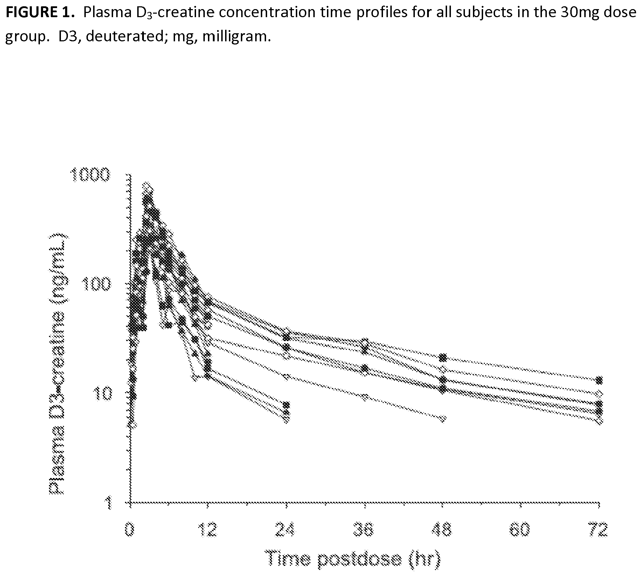

FIGURE 1 shows the plasma $D_3$-creatine concentration time profiles for all subjects in the 30mg dose group. D3, deuterated; mg, milligram.

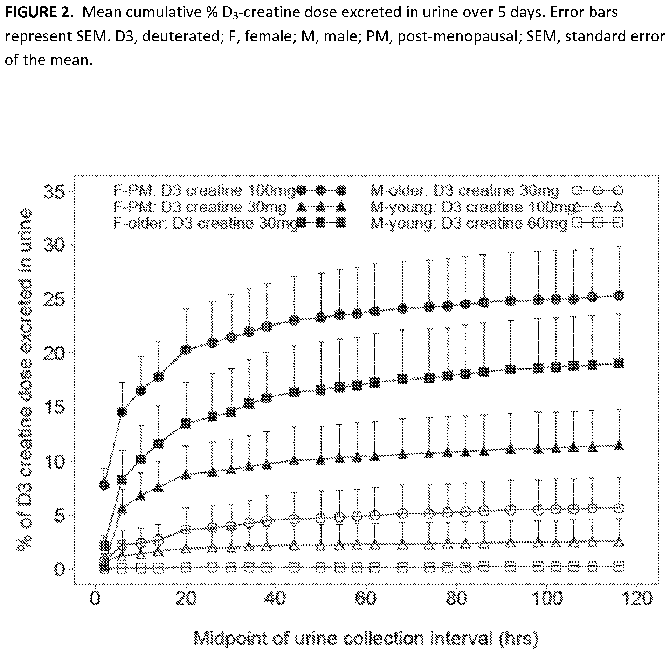

FIGURE 2 shows the mean cumulative % $D_3$-creatine dose excreted in urine over 5 days. Error bars represent SEM. D3, deuterated; F, female; M, male; PM, post-menopausal; SEM, standard error of the mean.

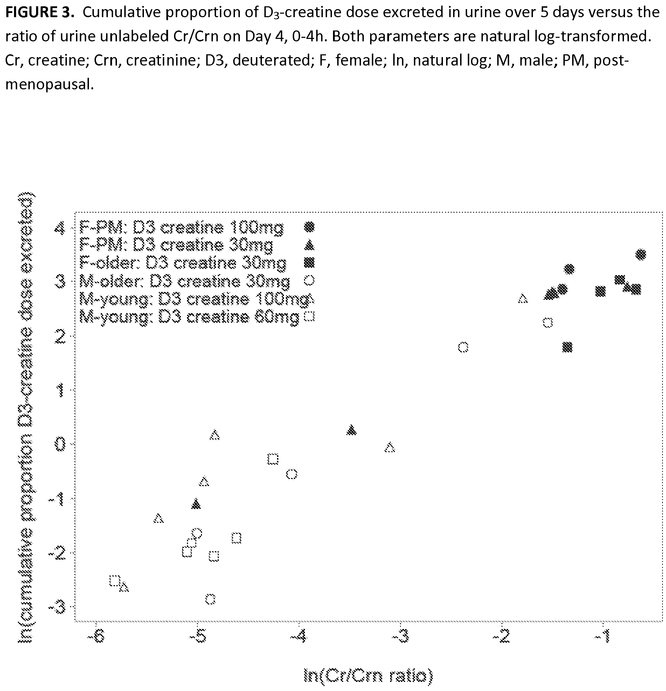

FIGURE 3 shows the cumulative proportion of $D_3$-creatine dose excreted in urine over 5 days versus the ratio of urine unlabeled Cr/Crn on Day 4, 0-4h. Both parameters are natural log-transformed. Cr, creatine; Crn, creatinine; D3, deuterated; F, female; In, natural log; M, male; PM, post-menopausal.

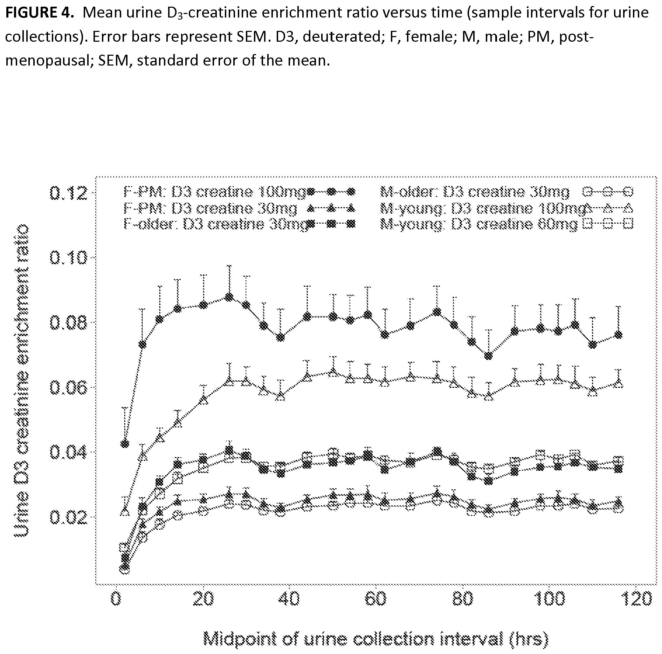

FIGURE 4 shows the mean urine $D_3$-creatinine enrichment ratio versus time (sample intervals for urine collections). Error bars represent SEM. D3, deuterated; F, female; M, male; PM, post-menopausal; SEM, standard error of the mean.

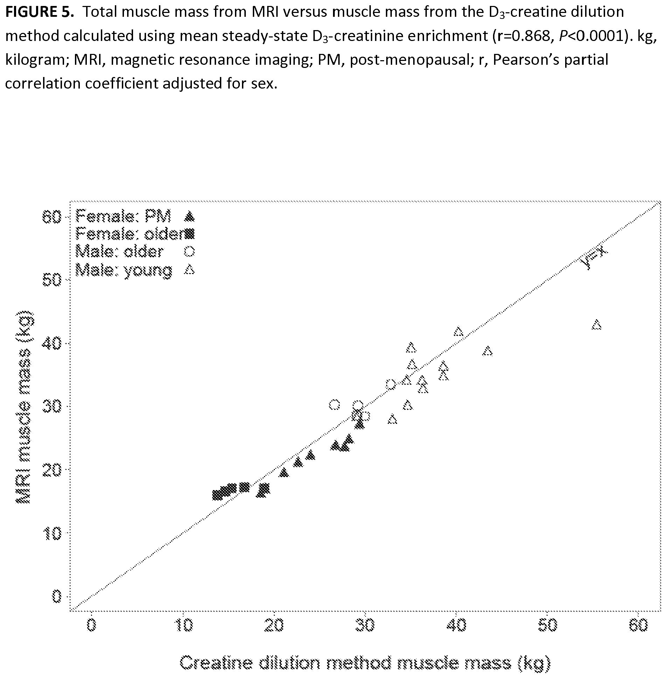

FIGURE 5 shows the total muscle mass from MRI versus muscle mass from the $D_3$-creatine dilution method calculated using mean steady-state $D_3$-creatinine enrichment (r=0.868, P<0.0001). kg, kilogram; MRI, magnetic resonance imaging; PM, post-menopausal; r, Pearson's partial correlation coefficient adjusted for sex.

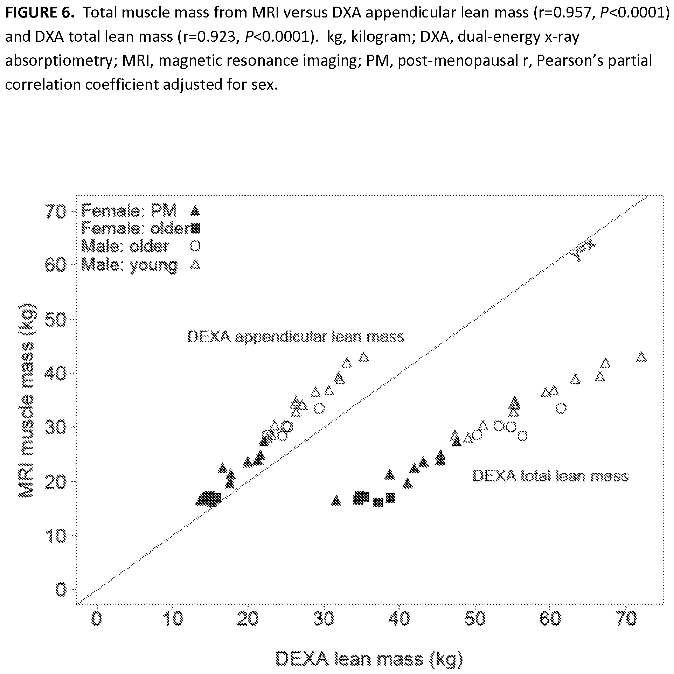

FIGURE 6 shows the total muscle mass from MRI versus DXA appendicular lean mass (r=0.957, P<0.0001) and DXA total lean mass (r=0.923, P<0.0001). kg, kilogram; DXA, dual-energy x-ray absorptiometry; MRI, magnetic resonance imaging; PM, post-menopausal r, Pearson's partial correlation coefficient adjusted for sex.

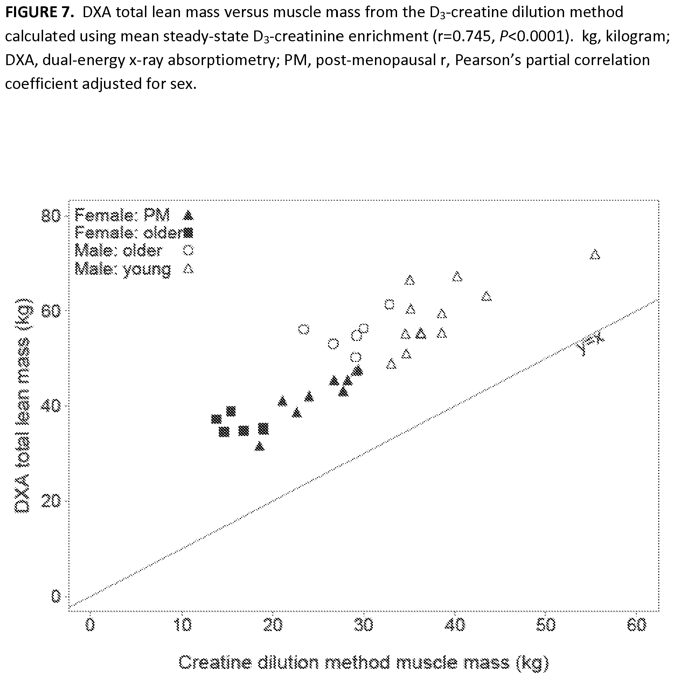

FIGURE 7 shows the DXA total lean mass versus muscle mass from the $D_3$-creatine dilution method calculated using mean steady-state $D_3$-creatinine enrichment (r=0.745, P<0.0001). kg, kilogram; DXA, dual-energy x-ray absorptiometry; PM, post-menopausal r, Pearson's partial correlation coefficient adjusted for sex.

DETAILED DESCRIPTION OF THE INVENTION

The present invention is based on the finding that enrichment of isotope-labeled creatinine in a urine sample following oral administration of a single defined dose of isotope-labeled creatine can be used to calculate total-body creatine pool size and skeletal muscle mass in a subject. Accordingly, the invention provides a non-invasive, accurate method of determining total body skeletal muscle. The methods of the invention find use, *inter alia*, in diagnosing and monitoring medical conditions associated with changes in total body skeletal muscle mass, and in screening potential therapeutic agents to determine their effects on muscle mass.

According to the method, isotope-labeled creatine is orally administered to a subject. Although the present is not limited by mechanism, it is believed that the isotope-labeled creatine is rapidly absorbed, distributed, and actively transported into skeletal muscle, where it is diluted in the skeletal muscle pool of creatine. Skeletal muscle contains the vast majority (> than 98%) of total-body creatine. In muscle tissue, creatine is converted to creatinine by an irreversible, non-enzymatic reaction at a stable rate of about 1.7% per day. This creatinine is a stable metabolite that rapidly diffuses from muscle, is not a substrate for the creatine transporter and cannot be transported back into muscle, and is excreted in urine. As a result, once an isotopic steady-state is reached, the enrichment of a isotope-labeled creatine in spot urine sample after a defined oral tracer dose of a isotope-labeled creatine reflects muscle creatine enrichment and can be used to directly determine creatine pool size. Skeletal muscle mass can then be calculated based on known muscle creatine content.

The present invention is based on the finding that steady-state enrichment of isotope-labeled creatinine in a urine sample following oral administration of a single defined tracer dose of isotope-labeled creatine can be used to calculate total-body creatine pool size and skeletal muscle mass in a subject.

In order to accurately determine the total-body creatine pool size of a subject according to the methods of the invention, it is necessary to determine the amount of the tracer dose of isotope-labeled creatine that is effectively delivered to the skeletal muscle of the subject. In order to calculate the amount of isotope-labeled creatine tracer dose that is effectively administered to the skeletal muscle of the subject, it is necessary to determine the amount of the administered isotope-labeled creatine tracer dose that is excreted into the urine of the subject rather than being delivered to the skeletal muscle of the subject. The inventors of the present invention have determined that the amount of the tracer dose of isotope-labeled creatine excreted into the urine can vary from subject to subject, depending on, *inter alia*, the body composition and diet of the subject. The inventors have further determined that the amount of the isotope-labeled creatine tracer dose that will be excreted into the urine is directly correlated with the creatine to creatinine ratio detected in a biological sample from the patient, and thus the ratio of creatine to creatinine in the biological sample from the patient can be used to accurately calculate the amount of the isotope-labeled creatine tracer dose that will be excreted into the urine.

Accordingly, in one aspect the invention provides a method for determining the total body skeletal muscle mass in a subject, where the method comprises the steps of:

(a) obtaining a first biological sample from the subject, wherein the first biological sample is a urine sample;
(b) determining the ratio of creatine to creatinine in the first biological sample from the subject;
(c) orally administering 20-100 mg isotope-labeled creatine or a salt or hydrate thereof to the subject;
(d) allowing at least 20 hours to elapse after the administration of the isotope-labeled creatine;
(e) obtaining a second biological sample from the subject;
(f) determining the concentration of creatinine and isotope-labeled creatinine in said second biological sample to thereby determine the isotope-labeled creatinine enrichment ratio in the second biological sample;
(g) using the creatine/creatinine ratio determined in step (b) to determine the amount of isotope-labeled creatine that has been effectively delivered to the skeletal muscle of the subject;
(h) calculating the total body skeletal muscle mass of the subject according to the formula:

Total body skeletal muscle mass = (amount of isotope-labeled creatine that has been effectively delivered to the skeletal muscle of the subject as determined according to step g)/[(the isotope-labeled creatinine enrichment ratio in the second biological sample as determined according to step (f)) X (the creatine content of skeletal muscle (g/kg))].

In a particular embodiment, the subject has fasted for at least 4 hours, at least 8 hours, or at least 12 hours before the first biological sample is obtained according to step (a).

According to the method, the second biological sample is preferably collected after enrichment levels of isotope-labeled creatinine in the second biological sample have reached a steady-state. Thus in one embodiment, at least 20 hours is allowed to elapse after the administration of the isotope-labeled creatine but prior to the collection of the biological sample. In certain embodiments, at least 24 hours is allowed to elapse. In particular embodiments, at least 30 hours, at least 36 hours, at least 40 hours, or at least 48 hours are allowed to elapse after the administration of the isotope-labeled creatine and before the collection of the biological sample.

In one embodiment, the second biological sample is a urine sample. In another embodiment, the second biological sample is a blood or serum sample. In an additional embodiment, the second biological sample is a muscle biopsy sample such as a micro biopsy sample.

In certain embodiments, a hydrate of isotope-labeled creatine is administered to the subject. In particular embodiments, isotope-labeled creatine monohydrate is administered. In other embodiments, isotope-labeled creatine anhydrate is administered.

The dose of isotope-labeled creatine to be administered to the subject is preferably selected such that the labeled creatine is rapidly absorbed into the bloodstream and excretion of excess label into the urine is minimized. Accordingly, for a human subject the dose of isotope-labeled creatine is typically 20-125 mgs. In particular embodiments, 5, 10, 20, 25, 30, 35, 40, 50, 60, 70, 80, 90, or 100 mgs of isotope-labeled creatine is administered. In certain embodiments, 10-50, such as 20-40, or more particularly, 30 mg of isotope-labeled creatine is administered to the subject. In other embodiments, 40-80 mg, such as 50-70, or more particularly, 60 mg or 70 mg of isotope-labeled creatine is administered to the subject.

The creatine to be administered may be labelled with any isotope label that does not interfere with the metabolism of creatine. By "isotope-labeled" is meant labeled with atoms with the same number of protons and hence of the same element but with different numbers of neutrons (e.g., $^1H$ vs. $^2H$). Isotope-labeled molecules are labeled with any possible isotope. Isotopes may be stable isotopes (e.g., $^2H$, $^{13}C$) or they may be radioisotopes (e.g., $^3H$, $^{14}C$). Examples of isotope-labeled creatine include $^2H_3$-creatine, $D_3$-creatine, $^{13}C_2$-creatine, $^{13}C_1$-creatine, or other species known in the art.

Pharmaceutical formulations adapted for oral administration may be presented as discrete units such as capsules or tablets; powders or granules; solutions or suspensions, each with aqueous or non-aqueous liquids; edible foams or whips; or oil-in-water liquid emulsions or water-in-oil liquid emulsions. For instance, for oral administration in the form of a tablet or capsule, the active drug component may be combined with an oral, non-toxic pharmaceutically acceptable inert carrier such as ethanol, glycerol, water, and the like. Generally, powders are prepared by comminuting the compound to a suitable fine size and mixing with an appropriate pharmaceutical carrier such as an edible carbohydrate, as, for example, starch or mannitol. Flavorings, preservatives, dispersing agents, and coloring agents may also be present.

Capsules can be made by preparing a powder, liquid, or suspension mixture and encapsulating with gelatin or some other appropriate shell material. Glidants and lubricants such as colloidal silica, talc, magnesium stearate, calcium stearate, or solid polyethylene glycol may be added to the mixture before the encapsulation. A disintegrating or solubilizing agent such as agar-agar, calcium carbonate or sodium carbonate may also be added to improve the availability of the medicament when the capsule is ingested. Moreover, when desired or necessary, suitable binders, lubricants, disintegrating agents, and coloring agents may also be incorporated into the mixture. Examples of suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, and the like. Lubricants useful in these dosage forms include, for example, sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

Tablets can be formulated, for example, by preparing a powder mixture, granulating or slugging, adding a lubricant and disintegrant, and pressing into tablets. A powder mixture may be prepared by mixing the compound, suitably comminuted, with a diluent or base as described above. Optional ingredients include binders such as carboxymethylcellulose, aliginates, gelatins, or polyvinyl pyrrolidone, solution retardants such as paraffin, resorption accelerators such as a quaternary salt, and/or absorption agents such as bentonite, kaolin, or dicalcium phosphate. The powder mixture may be wet-granulated with a binder such as syrup, starch paste, acadia mucilage or solutions of cellulosic or polymeric materials, and forcing through a screen. As an alternative to granulating, the powder mixture may be run through the tablet machine and the result is imperfectly formed slugs broken into granules. The granules may be lubricated to prevent sticking to the tablet forming dies by means of the addition of stearic acid, a stearate salt, talc or mineral oil. The lubricated mixture is then compressed into tablets. The compounds of the present invention may also be combined with a free flowing inert carrier and compressed into tablets directly without going through the granulating or slugging steps. A clear or opaque protective coating consisting of a sealing coat of shellac, a coating of sugar or polymeric material, and a polish coating of wax may be provided. Dyestuffs may be added to these coatings to distinguish different unit dosages.

Oral fluids such as solutions, syrups, and elixirs may be prepared in dosage unit form so that a given quantity contains a predetermined amount of the compound. Syrups may be prepared, for example, by dissolving the compound in a suitably flavored aqueous solution, while elixirs are prepared through the use of a non-toxic alcoholic vehicle. Suspensions may be formulated generally by dispersing the compound in a non-toxic vehicle. Solubilizers and emulsifiers such as ethoxylated isostearyl alcohols and polyoxy ethylene sorbitol ethers may be added. Solubilizers that may be used according to the present invention include Cremophor EL, vitamin E, PEG, and Solutol. Preservatives and/or flavor additives such as peppermint oil, or natural sweeteners, saccharin, or other artificial sweeteners; and the like may also be added.

The detection of creatine, creatinine, and isotope-labeled creatinine in biological samples can be performed according to methods known in the art, for example LC/MS/MS (see, for example, PCT/US2012/068068), direct or indirect colorimetric measurements, the Jaffé method, enzymatic degradation analysis, or derivatization of the creatinine followed by GC/MS analysis of HPLC with fluorescence detection.

The biological sample may be any appropriate sample including, but not limited to, urine, blood, serum, plasma, or tissue. In one particular embodiment, the biological sample is a urine sample. In another particular embodiment, the biological sample is a blood sample.

The methods of the invention are useful for diagnosing and monitoring medical conditions associated with changes in total body skeletal muscle mass. Examples of medical conditions in which loss of muscle mass plays an important role in function, performance status, or survival include, but are not limited to frailty and sarcopenia in the elderly; cachexia (e.g., associated with cancer, chronic obstructive pulmonary disease (COPD), heart failure, HIV-infection, tuberculosis, end stage renal disease (ESRD); muscle wasting associated with HIV therapy, disorders involving mobility disability (e.g., arthritis, chronic lung disease); neuromuscular diseases (e.g., stroke, amyotrophic lateral sclerosis); rehabilitation after trauma, surgery (including hip-replacement surgery), medical illnesses or other conditions requiring bed-rest; recovery from catabolic illnesses such as infectious or neoplastic conditions; metabolic or hormonal disorders (e.g., diabetes mellitus, hypogonadal states, thyroid disease); response to medications (e.g., glucocorticoids, thyroid hormone); malnutrition or voluntary weight loss. The claimed methods are also useful in sports-related assessments of total body skeletal muscle mass.

The methods of the invention are also useful for screening test compounds to identify therapeutic compounds that increase total body skeletal muscle mass. According to this embodiment, the total body skeletal mass of a subject is measured according to the method before and after a test compound is administered to the subject. The assessment of total body skeletal muscle mass can be repeated at appropriate intervals to monitor the effect of the test compound on total body skeletal muscle mass.

The following examples are intended for illustration only and are not intended to limit the scope of the invention in any way.


EXPERIMENTAL


**Clinical Validation a method to estimate muscle mass using a tracer dose of $D_3$-creatine that results in isotopic enrichment of $D_3$-creatinine**


For the clinical studies, thirty five healthy subjects were enrolled (33 completed) from diverse groups to provide a range of muscle mass: 13 young men (18-30yr), 10 post-menopausal women (50-60yr), 7 older men and 5 older women (70-85yr). Subjects were housed on the in-patient unit for the full 5 day study. Subjects were admitted on Day -1, for baseline evaluation and acclimation. After an overnight fast, subjects were given a single oral dose of 30, 60, or 100mg of $D_3$-creatine at 8AM on Day 1, and followed for blood and urine sampling for 5 days.

Serial plasma samples were collected for measurement of $D_3$-creatine for the first 12 h after dose in all subjects (0.25, 0.5, 1.0, 1.5, 2.0, 2.5, 3, 4, 5, 6, 8, 10, and 12 hr). In the older subjects, sampling was extended to 72 hr (24, 36, 48, and 72hr). Urine was collected continuously beginning at baseline, day -1, through day 5 of the study in timed intervals. On Day -1, subjects entered the clinic prior to noon, and timed urine collections began at 12:00PM with 12:00PM being time 0 (0-4, 4-8, 8-12, 12-20 h), and completed at 8:00AM the next day, Day 1. Urine samples were collected on Days 1-5 beginning at 8:00AM, time 0 (0-4, 4-8, 8-12, 12-16 and 16-24 h).

Initially, the tracer dose of $D_3$-creatine was 100mg in 7 of the young men and 6 of the post-menopausal women. Subsequently, doses of 60mg in 6 of the young men, 30mg in 4 post-menopausal women and 30mg in all the older men and women were administered. Of the 35 subjects enrolled, all received a single oral dose of $D_3$-creatine, and 33 subjects completed the study. 2 subjects did not complete the study.

On day 1, following the single oral dose of $D_3$-creatine at 8:00 am, breakfast was provided at 10 am. Subsequently, meals were provided for breakfast at 8:00 am, lunch at 1:00 pm, and dinner at 6:00 pm. Calorie content of meals was: 25% in breakfast, 30% in lunch, and 45% in dinner, with a macronutrient content of 45% carbohydrate, 35% fat, and 25% protein (animal and vegetable).

Measurement of plasma $D_3$-creatine, and urine $D_3$-creatine, $D_3$- creatinine and unlabeled creatine and creatinine, was done by liquid chromatography/mass spectrometry (LC/MS/MS) essentially as described in PCT/US2012/068068. Total body creatine pool size and muscle mass were calculated from $D_3$-creatinine enrichment in urine. Total body muscle mass was measured by MRI (serial cross sections), and total lean body mass (LBM) and appendicular lean mass (ALM) were measured by DXA during the subjects' stay on the inpatient unit. Muscle mass was also estimated from 24h urine creatinine excretion from the in-house urine collections. See Heymsfield et al. (1983) Am J Clin Nutr 37(3):478-94 and Arteaga C, McManus C, Smith J, Moffitt S. Measurement of muscle mass in humans:

validity of the 24-hour urinary creatinine method. Am J Clin Nutr 1983;37(3):478-94, and Wang et al. (1996) Am J Clin Nutr 63:863-9.

*Pharmacokinetic Methods*

Pharmacokinetic analysis of $D_3$-creatine plasma concentration-time data was done using noncompartmental analysis (Phoenix 6.3 WinNonlin 6.3 Pharsight, Certara Company). The PK parameters included the area under the plasma concentration vs. time curve from time zero to 24 hours AUC(0-24)and extrapolation to AUC(0-∞),-maximum observed plasma concentration (Cmax), Tmax, and T½ (initial and terminal), In addition, the systemic clearance (CLs) of $D_3$-creatine was calculated as dose divided by AUC(0-∞) and renal clearance of $D_3$-creatine (CLr) was calculated as the amount of $D_3$-creatine excreted in the first 24 hours post-dose divided by the plasma AUC(0-24).

*Statistical Methods*

The data were analyzed in SAS v9.2 (SAS Institute, Cary, NC) and graphs were produced in either SAS or TSCG. Results are presented as mean ± standard deviation (SD) unless noted otherwise. There were no adjustments for multiplicity.

The cumulative amount of $D_3$-creatine in urine represents the amount of the dose not taken up in the body creatine pool. For use in the calculation of creatine pool size and muscle mass, the cumulative amount of $D_3$-creatine excreted in urine and percent of dose excreted over 120 hrs post-dose was calculated.

Enrichment of $D_3$-creatinine in urine to total creatinine in urine was determined for each urine collection interval. Achievement of steady-state enrichment was determined by a combination of visual inspection and linear regression. To allow for complete distribution of the $D_3$-creatine dose and for the enrichment ratio to reach its maximum, the first 24hs of urine collections post-dose were excluded from assessment of steady-state. Linear regression of the enrichment ratio on time (midpoint of the urine collection interval) was performed for each subject separately. If the slope was statistically significantly different from 0 using alpha=0.10, the earliest time point was dropped and the regression performed again. This process was repeated until the slope was not statistically significantly different from 0. The earliest time point included in the final regression was defined as the time to achievement of steady-state. The mean enrichment ratio during steady-state was used in the calculation of creatine pool size and muscle mass.

$$\text{Creatine pool size (g)} = \frac{D_3\text{-Cr dose (mg)} - 120 \text{ hour cumulative amount (mg) of urine } D_3\text{-Cr}}{1000 \times \text{enrichment ratio}}$$

$$\text{Muscle mass (kg)} = \frac{\text{creatine pool size(g)}}{4.3 \text{ g/kg}}$$

where 4.3g/kg represents the creatine concentration in whole wet muscle mass. See, for example, Kreisberg et al. (1970) J Appl Physiol 28:264-7.

Muscle mass was also estimated from 24hr urine creatinine excretion. For each subject, the mean muscle mass from the first 3 days of urine collections was reported. Estimates of the fractional turnover rate (K) used in the equation ranged from 0.014-0.018. A value of 0.0169 was used for all subjects in this study. Although this estimate of K appears to be a reasonable estimate for healthy young men, it is unknown whether it is appropriate for post-menopausal women and older subjects.

Linear regression and Pearson's correlation coefficients were used to examine linear relationships between methods of estimating muscle mass and the strength of the relationship. Because clustering of groups can artificially inflate correlation coefficients, partial correlation coefficients adjusted for sex were computed.

To assess the agreement between MRI and the creatine dilution method and between MRI and DXA total lean mass, plots of the difference between the two methods versus the mean of the two methods were produced using a method to ascertain agreement

*Pharmacokinetics of $D_3$-creatine*

The $D_3$-creatine was rapidly absorbed and cleared from plasma (~80%) within 24 h of the oral tracer dose and essentially cleared by 72 h. Blood levels of $D_3$-creatine were observed at 15 minutes following a single dose with peak plasma concentrations by 2.5-3.0 h. $D_3$-creatine concentration time profiles for all subjects in the 30mg dose group are shown in **Figure 1**. When blood was sampled up to 72 h post dose, plasma concentrations were measurable at varying times out to 72 hours, below or near the lower limits of quantitation, (5 ng/mL).

Comparisons of Cmax and AUC(0-24) between low and high doses in the postmenopausal women and young men indicate dose proportionality leading to the conclusion that there was no tracer dose effect and 30mg to 100mg is an appropriate dose range for use in our method. The mean initial t1/2 parameter representing the first phase of decline of the plasma concentration profiles ranged from 2.7 to 3.4 h across all groups. These values are similar to the t1/2 values previously reported (Persky, 2003a). The mean terminal t1/2, a second phase of clearance and redistribution, ranged from 12.5 to 22 h, and this was measured only in the older group of men and women. The % of D3-creatine recovered in urine over 120 h ranged from 0.1 to 34% reflecting a broad range of renal clearance that is related to both age and sex. Renal clearance varied, ranging from 0.05% to 26% of the dose, or 0.26 to 56 mL/min . The renal excretion was lowest in young men with only 1 of 13 subjects excreting more than 2% of the dose over 5 days. In contrast, half of the older men (3/6) excreted >5% of the dose in urine. The women excreted the greatest percent of their dose in urine (medians: 16.1% in the post-menopausal women and 25.3% in the older women). And the majority of post-menopausal women (7 of 9) and all of the older women excreted >5%. In all subjects, the majority of the $D_3$-creatine excreted in urine occurred in the first 24hrs post-dose (**Figure 2**).

In an effort to understand $D_3$-creatine urinary excretion, the relationship was examined between the cumulative excretion of $D_3$-creatine after 5 days and the ratio of urine unlabeled creatine to urine unlabeled creatinine (Cr/Crn). **Figure 3** shows a log-log plot of the cumulative proportion of the $D_3$-creatine dose excreted after 5 days is positively related to the ratio of Cr/Crn (correlation (r)=0.924, P-value (P)<0.0001). The Cr/Crn ratio in the figure is from Day 4 (0-4h) and was selected to be in the same time frame as steady-state $D_3$-creatinine enrichment. This relationship also exists when other collection intervals for Cr/Crn are examined.

*Isotopic Enrichment of Urine*

The mean enrichment ratio for each group is plotted over time in **Figure 4**. By comparing enrichment between sexes in the same dose group, enrichment was greater in PMW than in young men and greater in older women than in older men. Less enrichment (greater dilution) of the tracer in the male subjects is consistent with our hypothesis and reflects a larger creatine pool size and greater muscle mass in men than in women.

The time to achievement of steady-state enrichment across all subjects was 30.7h $\pm$ 11.33h. Steady-state was observed in the majority of subjects (67%) by 24-28h post-dose with another 24% achieving steady-state by 32-36h. It took considerably longer (56-76h) for 3 of the women to achieve steady-state.

*Comparison of Methods*

The enrichment method estimates of creatine pool size and muscle mass or lean mass estimates from all methods are summarized in **Table 1**. Creatine pool size estimates ranged from a low of 68.5 $\pm$ 8.6g in the older women to a high of 162.2 $\pm$ 27.5g in the young men. Similarly, muscle mass estimates from all methods are lowest in older women, followed by post-menopausal women and older men with highest estimates in young men.

**Table 1. Summary of creatine pool size (g) by $D_3$-creatine dilution method and muscle mass estimates (kg) by all methods**

| Parameter | PMW[1] | Older Women | Young Men | Older Men |
|---|---|---|---|---|
| N | 9 | 5 | 13 | 6 |
| Enrichment method creatine pool size (g) | 103.9 (17.58)[2] | 68.5 (8.62) | 162.2 (27.47) | 122.7 (13.78) |
| Enrichment method muscle mass (kg) | 24.2 (4.09) | 15.9 (2.0) | 37.7 (6.39) | 28.5 (3.21) |
| MRI muscle mass (kg) | 21.9 (3.62) | 16.8 (0.52) | 35.4 (4.73) | 30.2 (2.03) |
| DEXA total lean mass (kg) | 41.1 (5.24) | 36.2 (1.79) | 58.3 (7.43) | 55.3 (3.7) |
| DEXA appendicular lean mass (kg) | 18.4 (2.98) | 15.0 (0.50) | 28.3 (4.12) | 25.3 (2.23) |

(continued)

| Parameter | PMW[1] | Older Women | Young Men | Older Men |
|---|---|---|---|---|
| 24h urine creatinine method muscle mass (kg) | 19.7 (4.08) | 13.0 (1.52) | 29.7 (3.96) | 24.0 (2.56) |
| [1]PMW = postmenopausal women [2] Mean $\pm$ SD (all such values) | | | | |

There was a strong correlation (r=0.868, p<0.0001) between MRI total muscle mass and the creatine dilution method estimate of muscle mass (**Figure 5**). Differences between MRI and dilution method estimates of muscle mass were plotted against the mean of the two methods (figure not shown). The mean difference (bias) between the two methods is low and indicates that the creatine dilution method overestimates MRI by 1.37kg. Approximately 95% of subjects could be expected to have a muscle measurement by MRI that is within 4.88kg greater than and 7.62kg less than that of the creatine dilution method. All subjects were combined for the Bland-Altman analysis but it is worth noting that the variability appears greater in the men than in the women (data not shown). Strong linear relationships also existed between MRI total muscle mass and both DXA total lean mass (r=0.923, P<0.0001) and appendicular lean mass (r=0.957, P<0.0001) (**Figure 6**). Relative to muscle mass determined by MRI, DXA total lean mass overestimated by (21.7kg $\pm$ 7.02 (mean $\pm$ 2SD) and DXA appendicular lean mass underestimated by (4.9kg $\pm$ 4.61 (mean $\pm$ 2SD). The correlation between muscle mass determined by the 24h urine creatinine method and MRI was 0.597 (P=0.0004). The correlation between DXA total lean mass and the creatine dilution method was 0.745 (*P*<0.0001) (**Figure 7**).

Isotopic steady state of urinary $D_3$-creatinine enrichment was demonstrated and was achieved by 30.7hr $\pm$ 11.2hr, and remained at steady state for the duration of the study, 120hr. The turnover of the total body creatine pool is slow and this allows for flexibility in the precise urine sampling time within a 3-5 day period after achievement of isotopic steady state. All subjects demonstrated a daily cyclic pattern of enrichment during this steady state period. Because there is no source of labeled creatinine other than skeletal muscle, the daily variability is most likely due to the consumption of food containing creatinine. Dietary creatinine is excreted in urine and would dilute the enrichment of labeled creatinine derived from intramuscular creatine. These data suggest that the use of a urine sample collected in the post absorptive state may reduce this variability.

The renal clearance of the tracer dose of $D_3$-creatinine was observed to be variable, ranging from 0.1% to 34% of the dose, or 0.26 to 56 mL/min. All urine was collected from each subject for the entire period of the study. In this way we were able to account for any $D_3$-creatine lost in urine, which allowed an accurate calculation of the creatine pool size. Large inter-subject variability in creatine excretion over 24hr was observed, leading to losses of orally delivered deuterium labeled creatine across the different groups of healthy subjects.

With one exception, the young men in this study showed minimal urinary losses of labeled creatine. However in the other subjects studied (post-menopausal women, older men and women), a greater amount of $D_3$-creatine in urine was observed, out to 72h. Approximately 75% of these subjects showed loss of the $D_3$-creatine tracer in urine exceeding 5% of the tracer dose. The increased excretion of the tracer dose in women and older subjects is a reflection of both renal clearance and circulating plasma levels of total creatine. There is no clear explanation for the differences in these populations. In this study, with complete collection of all urine for the full observational period, the loss of the tracer dose was determined and used for correction of the estimation of the creatine pool size.

**Figure 3** shows the relationship between the loss of the $D_3$-creatine tracer to the ratio of creatine/creatinine. The positive nature of the correlation indicates that measuring this ratio provides a correction for the loss of the D3-creatine tracer in urine. For example, the men in this study who had only trivial loss of $D_3$ creatine also showed the lowest creatine/creatinine ratio. Thus, the use of this ratio in a fasting 4hr urine collection as a means of correcting for urinary excretion of $D_3$-creatine dose to eliminate the need for extended urine collections to measure tracer dose recovery.

**Claims**

1. A method for determining the total body skeletal muscle mass in a subject, where the method comprises the steps of:

    (a) obtaining a first biological sample from the subject, wherein the first biological sample is a urine sample;
    (b) determining the ratio of creatine to creatinine in the first biological sample from the subject;
    (c) orally administering isotope-labeled creatine or a salt or hydrate thereof to the subject;
    (d) allowing the isotope-labeled creatine to reach isotopic steady state;
    (e) obtaining a second biological sample from the subject,

(f) determining the concentration of creatinine and isotope-labeled creatinine in said second biological sample to thereby determine the isotope-labeled creatinine enrichment ratio in the second biological sample;
(g) using the creatine/creatinine ratio determined in step (b) to determine the amount of isotope-labeled creatine that has been effectively delivered to the skeletal muscle of the subject;
(h) calculating the total body skeletal muscle mass of the subject according to the formula:

Total body skeletal muscle mass = (amount of isotope-labeled creatine that has been effectively delivered to the skeletal muscle of the subject as determined according to step g)/ [ (the isotope-labeled-creatinine enrichment ratio in the second biological sample as determined according to step (f)) X (creatine content of skeletal muscle (g/kg))].

2. The method according to claim 1 wherein the subject has fasted for at least 4 hours before the first biological sample is obtained according to step (a).

3. The method of claim 1 or 2, wherein said second biological sample is a urine sample.

4. The method of claim 1 or 2, wherein said second biological sample is a blood sample.

5. The method of any one of claims 1-4 wherein 10-50 mg of isotope-labeled creatine is administered to the subject.

6. The method of any one of claims 1-4 wherein 20-40 mg of isotope-labeled creatine is administered to the subject.

7. The method of any one of claims 1-6, wherein the second biological sample is obtained after at least 30 hours have elapsed after the administration of the isotope-labeled creatine.

8. The method of any one of claims 1 to 7, wherein the creatine content of skeletal muscle is estimated to be 4.3 g/kg.

9. The method of any one of claims 1 to 8, wherein the isotope-labeled creatine is D3-creatine and the isotope-labeled creatinine is $D_3$-creatinine.

10. A method for determining the total body skeletal muscle mass in a subject, where the method comprises the steps of:

(a) orally administering isotope-labeled creatine or a salt or hydrate thereof to the subject;
(b) allowing the isotope-labeled creatine to reach isotopic steady state;
(c) obtaining a biological sample from the subject,
(d) determining the ratio of creatine to creatinine in the biological sample from the subject.
(e) determining the concentration of creatinine and isotope-labeled creatinine in said biological sample to thereby determine the isotope-labeled creatinine enrichment ratio in the biological sample;
(f) using the creatine/creatinine ratio determined in step (d) to determine the amount of isotope-labeled creatine that has been effectively delivered to the skeletal muscle of the subject;
(g) calculating the total body skeletal muscle mass of the subject according to the formula:

Total body skeletal muscle mass = (amount of isotope-labeled creatine that has been effectively delivered to the skeletal muscle of the subject as determined according to step f)/ [ (the isotope-labeled creatinine enrichment ratio in the biological sample as determined according to step (e)) X (creatine content of skeletal muscle (g/kg))].

**Patentansprüche**

1. Verfahren zum Bestimmen der Gesamtkörperskelettmuskelmasse eines Individuums, wobei das Verfahren folgende Schritte umfasst:

   (a) Erhalten einer ersten biologischen Probe des Individuums, wobei die erste biologische Probe eine Urinprobe ist;
   (b) Bestimmen des Verhältnisses von Kreatin zu Kreatinin in der ersten biologischen Probe des Individuums;
   (c) orales Verabreichen von isotopenmarkiertem Kreatin oder eines Salzes oder Hydrates davon an das Individuum;
   (d) Zulassen, dass das isotopenmarkierte Kreatin ein isotopisches Fließgleichgewicht erlangt;
   (e) Erhalten einer zweiten biologischen Probe des Individuums,
   (f) Bestimmen der Konzentration von Kreatinin und isotopenmarkiertem Kreatinin in der zweiten biologischen Probe, um dadurch das Anreicherungsverhältnis von isotopenmarkiertem Kreatinin in der zweiten biologischen Probe zu bestimmen;
   (g) Verwenden des in Schritt b) bestimmten Kreatin/Kreatinin-Verhältnisses zum Bestimmen der Menge an isotopenmarkiertem Kreatin, die tatsächlich der Skelettmuskulatur des Individuums zugeführt wurde;
   (h) Berechnen der Gesamtkörperskelettmuskelmasse des Individuums gemäß der Formel:

   Gesamtkörperskelettmuskelmasse = (Menge an isotopenmarkiertem Kreatin, die tatsächlich der Skelettmuskulatur des Individuums zugeführt wurde, bestimmt gemäß Schritt g)/ [(Anreicherungsverhältnis von isotopenmarkiertem Kreatinin in der zweiten biologischen Probe, bestimmt gemäß Schritt (f)) X (Kreatingehalt der Skelettmuskulatur (g/kg))].

2. Verfahren nach Anspruch 1, wobei das Individuum mindestens vier Stunden lang vor dem Erhalten der biologischen Probe gemäß Schritt (a) gefastet hat.

3. Verfahren nach Anspruch 1 oder 2, wobei die zweite biologische Probe eine Urinprobe ist.

4. Verfahren nach Anspruch 1 oder 2, wobei die zweite biologische Probe eine Blutprobe ist.

5. Verfahren nach einem der Ansprüche 1-4, wobei dem Individuum 10-50 mg isotopenmarkiertes Kreatin verabreicht werden.

6. Verfahren nach einem der Ansprüche 1-4, wobei dem Individuum 20-40 mg isotopenmarkiertes Kreatin verabreicht werden.

7. Verfahren nach einem der Ansprüche 1-6, wobei die zweite biologische Probe nach Ablauf von mindestens 30 Stunden ab der Verabreichung des isotopenmarkierten Kreatins erhalten wird.

8. Verfahren nach einem der Ansprüche 1-7, wobei der Kreatingehalt der Skelettmuskulatur auf 4,3 g/kg geschätzt wird.

9. Verfahren nach einem der Ansprüche 1-8, wobei das isotopenmarkierte Kreatin D3-Kreatin und das isotopenmarkierte Kreatinin $D_3$-Kreatinin ist.

10. Verfahren zum Bestimmen der Gesamtkörperskelettmuskelmasse eines Individuums, wobei das Verfahren folgende Schritte umfasst:

    (a) orales Verabreichen von isotopenmarkiertem Kreatin oder eines Salzes oder Hydrates davon an das Individuum;
    (b) Zulassen, dass das isotopenmarkierte Kreatin ein isotopisches Fließgleichgewicht erlangt;
    (c) Erhalten einer biologischen Probe des Individuums,
    (d) Bestimmen des Verhältnisses von Kreatin zu Kreatinin in der biologischen Probe des Individuums,
    (e) Bestimmen der Konzentration von Kreatinin und isotopenmarkiertem Kreatinin in der biologischen Probe, um dadurch das Anreicherungsverhältnis von isotopenmarkiertem Kreatinin in der biologischen Probe zu bestimmen;
    (f) Verwenden des in Schritt (d) bestimmten Kreatin/Kreatinin-Verhältnisses zum Bestimmen der Menge an isotopenmarkiertem Kreatin, die tatsächlich der Skelettmuskulatur des Individuums zugeführt wurde;

(g) Berechnen der Gesamtkörperskelettmuskelmasse des Individuums gemäß der Formel:

Gesamtkörperskelettmuskelmasse = (Menge an isotopenmarkiertem Kreatin, die tatsächlich der Skelettmuskulatur des Individuums zugeführt wurde, bestimmt gemäß Schritt f)/ [(Anreicherungsverhältnis von isotopenmarkiertem Kreatinin in der biologischen Probe, bestimmt gemäß Schritt (e)) X (Kreatingehalt der Skelettmuskulatur (g/kg))].

**Revendications**

1. Procédé de détermination de la masse musculaire squelettique totale du corps chez un sujet, ledit procédé comprenant les étapes consistant à:

   (a) obtenir un premier échantillon biologique provenant du sujet, le premier échantillon biologique étant un échantillon d'urine;
   (b) déterminer le rapport de la créatine en créatinine dans le premier échantillon biologique provenant du sujet;
   (c) administrer, au sujet par voie orale, une créatine marquée par un isotope, ou un sel ou un hydrate de celui-ci;
   (d) permettre à la créatine marquée par un isotope d'atteindre l'état d'équilibre isotopique;
   (e) obtenir un deuxième échantillon biologique provenant du sujet,
   (f) déterminer la concentration de créatinine et de créatinine marquée par un isotope dans ledit deuxième échantillon biologique afin de déterminer ainsi le rapport d'enrichissement de créatinine marquée par un isotope dans le deuxième échantillon biologique;
   (g) utiliser le rapport de créatine/créatinine déterminé à l'étape (b) pour déterminer la quantité de créatine marquée par un isotope qui a été effectivement délivrée au muscle squelettique du sujet;
   (h) calculer la masse musculaire squelettique totale du corps conformément à la formule:

   masse musculaire squelettique totale du corps = (quantité de créatine marquée par un isotope qui a été effectivement délivrée au muscle squelettique du sujet tel que déterminée conformément à l'étape g)/ [(le rapport d'enrichissement de créatine marquée par un isotope dans le deuxième échantillon biologique tel que déterminé selon l'étape (f)) X (teneur en créatine de muscle squelettique (g/kg))].

2. Procédé selon la revendication 1, dans lequel le sujet a jeûné pendant au moins 4 heures avant l'obtention du premier échantillon biologique selon l'étape (a).

3. Procédé selon la revendication 1 ou 2, dans lequel ledit deuxième échantillon biologique est un échantillon d'urine.

4. Procédé selon la revendication 1 ou 2, dans lequel ledit deuxième échantillon biologique est un échantillon de sang.

5. Procédé selon l'une quelconque des revendications 1 à 4, dans lequel une quantité de 10 à 50 mg de créatine marquée par un isotope est administrée au sujet.

6. Procédé selon l'une quelconque des revendications 1 à 4, dans lequel une quantité de 20 à 40 mg de créatine marquée par un isotope est administrée au sujet.

7. Procédé selon l'une quelconque des revendications 1 à 6, dans lequel le deuxième échantillon biologique est obtenu après qu'au moins 30 heures se sont écoulées après l'administration de la créatine marquée par un isotope.

8. Procédé selon l'une quelconque des revendications 1 à 7, dans lequel la teneur en créatine du muscle squelettique est estimée à 4,3 g/kg.

9. Procédé selon l'une quelconque des revendications 1 à 8, dans lequel la créatine marquée par un isotope est la créatine D3, et la créatinine marquée par un isotope est la créatinine $D_3$.

**10.** Procédé de détermination de la masse musculaire squelettique totale du corps chez un sujet, ledit procédé comprenant les étapes consistant à:

(a) administrer, au sujet par voie orale, une créatine marquée par un isotope, ou un sel ou un hydrate de celle-ci;
(b) permettre à la créatine marquée par un isotope d'atteindre l'état d'équilibre isotopique;
(c) obtenir un échantillon biologique provenant du sujet,
(d) déterminer le rapport de la créatine en créatinine dans l'échantillon biologique provenant du sujet;
(e) déterminer la concentration de créatinine et de créatinine marquée par un isotope dans ledit échantillon biologique afin de déterminer ainsi le rapport d'enrichissement de créatinine marquée par un isotope dans l'échantillon biologique;
(f) utiliser le rapport de créatine/créatinine déterminé à l'étape (d) pour déterminer la quantité de créatine marquée par un isotope qui a été effectivement délivrée au muscle squelettique du sujet;
(g) calculer la masse musculaire squelettique totale du corps conformément à la formule:

masse musculaire squelettique totale du corps = (quantité de créatine marquée par un isotope qui a été effectivement délivrée au muscle squelettique du sujet tel que déterminée conformément à l'étape f) / [(le rapport d'enrichissement de créatine marquée par un isotope dans l'échantillon biologique tel que déterminé selon l'étape (e)) X (teneur en créatine de muscle squelettique (g/kg))].

**FIGURE 1.** Plasma D$_3$-creatine concentration time profiles for all subjects in the 30mg dose group.  D3, deuterated; mg, milligram.

**FIGURE 2.** Mean cumulative % D$_3$-creatine dose excreted in urine over 5 days. Error bars represent SEM. D3, deuterated; F, female; M, male; PM, post-menopausal; SEM, standard error of the mean.

**FIGURE 3.** Cumulative proportion of $D_3$-creatine dose excreted in urine over 5 days versus the ratio of urine unlabeled Cr/Crn on Day 4, 0-4h. Both parameters are natural log-transformed. Cr, creatine; Crn, creatinine; D3, deuterated; F, female; ln, natural log; M, male; PM, post-menopausal.

**FIGURE 4.** Mean urine $D_3$-creatinine enrichment ratio versus time (sample intervals for urine collections). Error bars represent SEM. D3, deuterated; F, female; M, male; PM, post-menopausal; SEM, standard error of the mean.

**FIGURE 5.** Total muscle mass from MRI versus muscle mass from the D$_3$-creatine dilution method calculated using mean steady-state D$_3$-creatinine enrichment (r=0.868, *P*<0.0001). kg, kilogram; MRI, magnetic resonance imaging; PM, post-menopausal; r, Pearson's partial correlation coefficient adjusted for sex.

**FIGURE 6.** Total muscle mass from MRI versus DXA appendicular lean mass (r=0.957, *P*<0.0001) and DXA total lean mass (r=0.923, *P*<0.0001). kg, kilogram; DXA, dual-energy x-ray absorptiometry; MRI, magnetic resonance imaging; PM, post-menopausal r, Pearson's partial correlation coefficient adjusted for sex.

**FIGURE 7.** DXA total lean mass versus muscle mass from the $D_3$-creatine dilution method calculated using mean steady-state $D_3$-creatinine enrichment (r=0.745, *P*<0.0001). kg, kilogram; DXA, dual-energy x-ray absorptiometry; PM, post-menopausal r, Pearson's partial correlation coefficient adjusted for sex.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012068068 W

**Non-patent literature cited in the description**

- **BARACOS et al.** *J. Parenter Enteral Nutr.,* 2012, vol. 36, 96-107
- **SARKAR et al.** *J. Ren. Nutr.,* 2005, vol. 15, 152-8
- **BALSOM et al.** *J. Sports Med.,* 1994, vol. 18, 268-80
- **CRIM et al.** *J. Nutr.,* vol. 105, 428-38
- **WYSS.** *Physiol. Rev.,* 2000, vol. 80, 1107-213
- **STIMPSON et al.** *J. Appl. Physiol.,* 2012, vol. 112, 1940-8
- **HEYMSFIELD et al.** *Am J Clin Nutr,* 1983, vol. 37 (3), 478-94
- **ARTEAGA C ; MCMANUS C ; SMITH J ; MOFFITT S.** Measurement of muscle mass in humans: validity of the 24-hour urinary creatinine method. *Am J Clin Nutr,* 1983, vol. 37 (3), 478-94
- **WANG et al.** *Am J Clin Nutr,* 1996, vol. 63, 863-9
- **KREISBERG et al.** *J Appl Physiol,* 1970, vol. 28, 264-7